# DEMANDE DE BREVET EUROPEEN

(11) **EP 3 916 732 A1**
(43) Date de publication de la demande: **01.12.2021**
(21) Numéro de dépôt: 20177011.2
(22) Date de dépôt: 28.05.2020
(51) Int. Cl.: G16H 20/40, A61M 13/00, A61M 16/00

(54) **INSTALLATION DE FOURNITURE D'OXYGÈNE INCLUANT UNE PLATEFORME INFORMATIQUE DE MANAGEMENT DE DONNÉES**

(71) Demandeur: Air Liquide Medical S.A., 4020 Liège (BE); L'Air Liquide Société Anonyme pour l'Etude et l'Exploitation des Procédés Georges Claude, 75007 Paris (FR)
(72) Inventeur: VAN DEN BOSSCHE, Jo, 1130 Brussels (BE)
(74) Mandataire: Air Liquide

(57) **Abrégé**

L'invention concerne une installation (1) de fourniture d'oxygène à un patient permettant d'assurer une surveillance et un suivi dudit patient à distance, en particulier un patient âgé de maison de retraite ou analogue infecté par des organismes pathogènes affectant ses capacités pulmonaires, par exemple une pathologie virale et/ou bactérienne, tel un coronavirus comme le virus Covid-19. Elle comprend une plateforme informatique de management de données (10) ; un dispositif de mesure de paramètres physiologiques (20) telle que la saturation en oxygène (SpO₂) et/ou la fréquence cardiaque du patient ; un débitmètre (30) communicant configuré pour fournir de l'oxygène gazeux au patient à un débit donné ; une source d'oxygène (40) ; un dispositif d'affichage de données (50) pour afficher des données fournies par la plateforme informatique (10) ; et des moyens de communication (60) configurés pour assurer une transmission de données entre les différents équipements.

## Description

L'invention concerne une installation de fourniture d'oxygène, notamment à des patients, notamment âgés, infectés par un ou des organismes pathogènes affectant ses capacités pulmonaires, par exemple une pathologie virale et/ou bactérienne, tel un coronavirus comme le virus Covid-19, laquelle installation permette d'assurer une surveillance et un suivi du patient à distance, y compris lorsqu'il se trouve dans une maison de retraite, de repos ou analogue, c'est-à-dire dans un environnement extrahospitalier.

Certaines pathologies ou insuffisances respiratoires nécessitent un traitement par oxygénothérapie visant à administrer de l'oxygène ou un gaz riche en oxygène aux patients en ayant besoin afin de faire remonter leur taux d'oxygène sanguin, c'est-à-dire leur SpO₂ ou taux de saturation en oxygène. Ceci est par exemple le cas de patients infectés par certains organismes pathogènes affectant les poumons, notamment des virus, en particulier des coronavirus, tel le virus Covid-19.

Si la pandémie liée au coronavirus appelé Covid-19 a impacté la capacité de nombreux hôpitaux à accueillir et donc soigner des patients infectés, il est rapidement apparu que les maisons de retraite, de repos ou analogue étaient encore plus impactées puisque le nombre de décès dans ces établissements est vite devenu bien plus élevé que dans les hôpitaux.

Une explication à cela est que les maisons de retraite, de repos ou analogue n'étaient pas équipées et/ou organisées pour fournir des soins médicaux poussés, tels des traitements d'oxygénothérapie, aux patients âgées infectés par le Covid-19 qui y résidaient. Ainsi, les maisons de retraite ne disposent pas toutes de réseaux de canalisations de distribution de gaz médicaux, comme c'est le cas dans les hôpitaux. Autrement dit, opérer des administrations d'oxygène suffisantes n'a souvent pas été possible au sein des maisons de retraites, de repos ou analogue, ce qui a conduit à des décès des patients les plus fragiles qui y résidaient.

D'une façon générale, dans les maisons de retraite, de repos ou analogue, les sources d'oxygène sont généralement limitées aux concentrateurs d'oxygène ou « oxyconcentrateurs », et aux bouteilles d'oxygène.

Les oxyconcentrateurs produisent un flux gazeux riche en oxygène à partir d'air ambient par séparation de l'azote qui s'y trouve, voire d'autres composés gazeux, tel qu'argon, CO₂, vapeur d'eau.... Toutefois, un inconvénient majeur de ces appareils est qu'ils fournissent de l'oxygène à débit trop bas et/ou à pureté limitée. Ainsi, le débit maximal produit est de l'ordre de 4 l/min pour une pureté de l'oxygène d'environ 95% (% en vol) ou de l'ordre de 5 l/min pour des puretés inférieures, ce qui est insuffisant pour beaucoup de patients infectés par le Covid-19 pour atteindre des niveaux de SpO₂ suffisamment élevés, c'est-à-dire pour faire remonter leur taux d'oxygène sanguin.

Par ailleurs, si les bouteilles d'oxygène peuvent fournir de l'oxygène pur (env. 100% de pureté) à des débits plus élevés, typiquement jusqu'à environ 15 l/min, donc à des pureté et débit suffisants pour traiter les patients âgés des maisons de retraite qui sont infectés par le Covid-19, celles-ci posent un problème d'autonomie. En effet, les bouteilles contiennent une quantité limitée d'oxygène qui est fonction de leur taille et de la quantité de gaz comprimé qui y est stockée. Une bouteille d'oxygène se videra donc d'autant plus vite que le débit d'oxygène demandé est élevé. En général, leur autonomie est comprise entre 1h et 24h environ, ce qui nécessite une logistique de (ré)approvisionnement, i.e. de remplacement des bouteilles vides par des bouteilles pleines, difficile à mettre en oeuvre lors d'une pandémie, c'est-à-dire lorsque des bouteilles d'oxygène doivent être livrées à de nombreux endroits différents (e.g. maisons de retraite, hôpitaux, tentes médicalisées...) dans un délai court, alors que les capacités de production et/ou les véhicules de livraison sont limités.

Afin de tenter de résoudre ces problèmes, il a été proposé d'utiliser des réservoirs transportables de stockage et de fourniture d'oxygène sous forme liquide de type Freelox^{™}, pouvant contenir plusieurs dizaines de litres d'oxygène liquide ou LOX. Si ceux-ci offrent des autonomies supérieures aux bouteilles et une meilleure pureté d'oxygène (i.e. env. 100%) que les oxyconcentrateurs, ils sont aussi limités quant au débit maximal d'oxygène pouvant être fourni et requièrent aussi une logistique de réapprovisionnement en LOX lorsqu'ils sont (presque) vides.

Par ailleurs, une autre problématique existante, quelle que soit la source d'oxygène utilisée, est celle du suivi du niveau de la SpO₂ des patients des maisons de retraite, de repos ou analogue étant donné que les personnels qui y travaillent ne sont pas des médecins ou des personnes habilitées à établir des diagnostics ou à prodiguer des soins.

Le problème est dès lors de proposer une installation de fourniture d'oxygène à des patients, notamment à des patients âgés, en particulier atteints infectés par des organismes pathogènes affectant ses capacités pulmonaires, par exemple une pathologie virale et/ou bactérienne, tel un coronavirus comme le virus Covid-19, de manière à résoudre tout ou partie des problématiques et inconvénients susmentionnés des sources d'oxygène utilisées en maisons de retraite, de repos ou analogue et donc à accroître la sécurité et le confort du patient tout en facilitant le travail du personnel des maisons de retraite, de repos ou analogue, tel un centre de soins spécialisé, par exemple un centre de soins pour personnes handicapées, un centre de soins psychiatriques ou autres.

La solution de l'invention concerne alors une installation de fourniture d'oxygène à un patient, en particulier un patient âgé et/ou atteint infecté par un ou des organismes pathogènes affectant ses capacités pulmonaires, par exemple une pathologie virale et/ou bactérienne, tel un coronavirus comme le virus Covid-19, permettant d'assurer une surveillance et suivi du patient à distance comprenant :
- une plateforme informatique de management de données configurée pour traiter des données,
- un dispositif de mesure de paramètres physiologiques du patient configuré pour mesurer au moins la saturation en oxygène (SpO₂) du patient,
- un débitmètre communicant configuré pour fournir de l'oxygène gazeux au patient à un débit donné, ledit débitmètre comprenant en outre des moyens de détermination de la fréquence respiratoire du patient et/ou de la quantité d'oxygène fournie au patient,
- une source d'oxygène relié fluidiquement au débitmètre communicant pour lui fournir de l'oxygène,
- un dispositif d'affichage de données configuré pour afficher des données fournies par la plateforme informatique de management de données, et
- des moyens de communication configurés pour assurer une ou des transmissions de données, i.e. des échanges de données, au moins :
   ∘ depuis le dispositif de mesure de paramètres physiologiques et le débitmètre communicant (i.e. directement ou indirectement) vers la plateforme informatique de management de données, et
   ∘ depuis la plateforme informatique de management de données vers le dispositif d'affichage de données.

Selon le mode de réalisation considéré, l'installation de l'invention peut comprendre l'une ou plusieurs des caractéristiques suivantes :
- le dispositif de mesure de paramètres physiologiques du patient configuré pour mesurer en outre la fréquence cardiaque du patient.
- la plateforme informatique de management de données est configurée pour traiter des mesures provenant du dispositif de mesure de paramètres physiologiques, en particulier SpO₂ et/ou la fréquence cardiaque du patient.
- la plateforme informatique de management de données est configurée pour :
   a) comparer des mesures de saturation en oxygène (i.e. SpO₂) provenant du dispositif de mesure de paramètres physiologiques à au moins une valeur-seuil haut et/ou une valeur-seuil bas de SpO₂ préfixée, et
   b) déclencher une alerte visuelle et/ou sonore lorsque la saturation en oxygène (SpO₂) mesurée descend en dessous (i.e. est inférieure à) ladite valeur-seuil bas de SpO₂ préfixée ou excède (i.e. est supérieure à) ladite valeur-seuil haut de SpO₂ préfixée.
- la valeur-seuil bas SpO₂ préfixée est égale à environ 92%, en particulier pour un patient atteint de Covid19.
- la valeur-seuil haut SpO₂ préfixée est égale à environ 98%, en particulier pour un patient atteint de Covid19.
- la plateforme informatique de management de données est configurée pour :
   a) comparer des mesures de rythme cardiaque provenant du dispositif de mesure de paramètres physiologiques à au moins une valeur-seuil de rythme cardiaque préfixée, et
   b) déclencher une alerte visuelle et/ou sonore lorsque le rythme cardiaque mesuré excède (i.e. est supérieure à) ladite valeur-seuil de rythme cardiaque préfixée.
- la valeur-seuil de rythme cardiaque préfixée est égale à environ 120 cts/min pour un patient atteint de Covid19.
- le dispositif de mesure de paramètres physiologiques comprend un saturomètre, de préférence un saturomètre connecté configuré pour émettre des données, i.e. une ou des mesures, en particulier en mode Bluetooth^{™} ou Wifi.
- le saturomètre est un saturomètre-bracelet ou une montre-bracelet intégrant un saturomètre ou un saturomètre classique de mesure au doigt du patient.
- le dispositif de mesure de paramètres physiologiques est configuré pour communiquer des données au débitmètre.
- le dispositif de mesure de paramètres physiologiques est configuré pour communiquer des données, notamment la SpO₂, directement à la plateforme informatique de management de données.
- le dispositif d'affichage de données comprend un ordinateur fixe ou portable, une tablette numérique ou un téléphone mobile multifonction (i.e. smartphone), de préférence il comprend un écran équipant l'un de ces équipements.
- le dispositif d'affichage de données est configuré pour afficher une alerte, notamment une alerte visuelle.
- les moyens de communication comprennent des moyens d'émission et/ou de réception de données, notamment en mode Bluetooth^{™}, Wifi, GSM, 4G/5G ou autre.
- les moyens de communication comprennent le réseau internet et/ou un réseau de téléphonie fixe ou mobile.
- la plateforme informatique de management de données comprend un (ou des) serveur informatique.
- la plateforme informatique de management de données comprend ou est reliée à des moyens de stockage de données, par exemple une ou des mémoires informatiques et/ou un (ou des) serveur de stockage de données.
- la source d'oxygène comprend un récipient d'oxygène sous pression, telle une bouteille de gaz, ou une canalisation ou tuyau d'amenée d'oxygène.
- la source d'oxygène comprend une canalisation d'amenée d'oxygène faisant partie d'un réseau de canalisations agencé dans un bâtiment, en particulier une maison de retraite, de repos ou analogue.
- le patient est une personne âgée d'au moins 60 ans, de préférence d'au moins 65 ans, voire d'au moins 70 ans.
- le patient est une personne pensionnaire ou résidente d'une maison de retraite, de repos ou analogue.
- le patient est un adulte ou un enfant, de préférence un adulte.
- le patient est dans un centre de soins spécialisé, par exemple un centre de soins pour personnes handicapées, un centre de soins psychiatriques ou autres.
- la source d'oxygène est ou comprend une bouteille de gaz sous pression équipée d'un robinet de distribution d'oxygène gazeux, de préférence un robinet à détendeur intégré (RDI).
- la bouteille de gaz est équipée d'un robinet de distribution d'oxygène gazeux équipé d'un dispositif électronique communicant de mesure de pression et/ou d'autonomie en gaz.
- la bouteille de gaz est équipée d'un robinet de distribution d'oxygène gazeux protégé par un capotage de protection, de préférence un capotage muni d'une poignée de transport et/ou d'un dispositif d'accrochage à un barreau de lit ou un autre support analogue, avantageusement un dispositif d'accrochage pivotant ou analogue.
- le débitmètre communicant est agencé entre la source d'oxygène et une interface respiratoire patient reliée à la source d'oxygène via au moins un conduit de gaz flexible.
- le débitmètre communicant comprend des moyens d'ajustement de débit pour ajuster ou changer le débit d'oxygène qu'il fournit au patient.
- le débitmètre communicant comprend en outre des moyens de calcul à microprocesseur, par exemple une carte électronique.
- les moyens d'ajustement de débit comprennent une vanne proportionnelle commandée par les moyens de calcul pour fixer ou ajuster le débit d'oxygène.
- le débitmètre comprend des moyens de détermination de la quantité d'oxygène fournie permettent de déterminer et fournir le débit d'oxygène fourni ou la quantité totale d'oxygène fournie pendant une durée donnée.
- l'interface respiratoire patient comprend un masque respiratoire nasal ou facial, ou des canules respiratoires. Un humidificateur à usage unique peut être utilisé pour saturer l'oxygène et le rendre moins sèche pour le confort du patient.
- le dispositif d'affichage de données comprend des moyens d'entrée de données permettant de rentrer, changer ou modifier une (ou des) donnée affichée ou un paramètre donné, par exemple de modifier à distance le débit d'oxygène délivré par le débitmètre, ou configurer une ou des alarmes ou alertes.
- les moyens d'entrée de données comprennent le clavier numérique d'une tablette numérique ou un téléphone mobile multifonction, ou le clavier mécanique d'un ordinateur fixe ou portable, ou d'autres dispositifs, telle une souris ou un écran tactile par exemple.
- la plateforme informatique de management de données est en outre configurée pour modifier ou corriger automatiquement le débit O₂ fourni par le débitmètre.

L'invention va maintenant être mieux comprise grâce à la description détaillée suivante, faite à titre illustratif mais non limitatif, en référence aux figures annexées parmi lesquelles :
- la Figure 1 schématise un premier mode de réalisation d'une installation de fourniture d'oxygène selon l'invention, et
- la Figure 2 schématise un deuxième mode de réalisation d'une installation de fourniture d'oxygène selon l'invention.

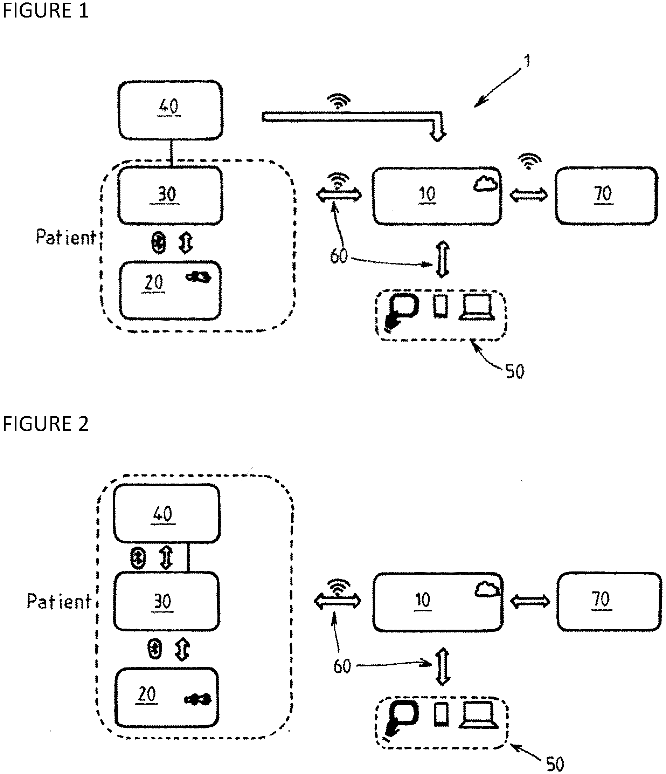

Les Figures 1 et 2 schématisent deux modes de réalisation d'une installation de fourniture d'oxygène selon l'invention. Dès lors, les mêmes références sont utilisées pour désigner des éléments identiques sur ces Figures et les explications données pour une Figure s'appliquent donc stricto sensu à l'autre, sauf indication contraire.

La Figure 1 schématise une installation 1 de fourniture d'oxygène à un patient permettant en outre d'assurer une surveillance et un suivi dudit patient à distance, en particulier un patient de maison de retraite ou analogue, atteint ou infecté par un ou des organismes pathogènes affectant ses capacités pulmonaires, par exemple une pathologie virale et/ou bactérienne, tel un coronavirus comme le virus Covid-19.

L'installation 1 comprend une plateforme informatique de management de données 10, tel un (ou des) serveur informatique, qui est configurée pour traiter des données, notamment des mesures qui lui sont fournies par les dispositifs de mesure 20, 30.

Ces dispositifs de mesure 20, 30 comprennent un dispositif de mesure de paramètres physiologiques 20 du patient et un débitmètre 30 communicant alimenté en oxygène par une source d'oxygène 40, qui est, dans le cas de la Figure 1, une canalisation d'amenée d'oxygène gazeux (pureté env. 100%) agencée dans le bâtiment où se trouve le patient, typiquement une maison de retraite.

Le dispositif de mesure de paramètres physiologiques 20 du patient est configuré pour mesurer la saturation en oxygène (SpO₂) du patient et préférentiellement aussi la fréquence cardiaque du patient. A cette fin, on peut utiliser un saturomètre, de préférence connecté communiquant, en particulier un saturomètre-bracelet ou une montre-bracelet connectée que le patient peut porter en permanence à son poignet ou, selon le cas, un saturomètre classique, aussi appelé oxymètre de pouls, permettant de réaliser les mesures au doigt du patient. Utiliser un saturomètre portatif, par exemple un saturomètre-bracelet ou une montre-bracelet intégrant un saturomètre, permet une surveillance continue de la SpO₂ et/ou du rythme cardiaque du patient de manière confortable pour le patient car sans que le patient ne s'en rende compte.

Le dispositif de mesure de paramètres physiologiques 20 comprend un boitier externe embarquant des moyens de mesure, notamment de SpO₂, et des moyens de calcul, ainsi qu'une source de courant, de préférence interne, telle une pile ou batterie, rechargeable ou à usage unique, qui l'alimente en courant électrique. Les moyens de calcul comprennent par exemple un microprocesseur, de préférence agencé sur une carte électronique. Le microprocesseur met en œuvre un ou des logiciels de calcul.

Le dispositif de mesure de paramètres physiologiques 20 du patient est préférentiellement connecté, c'est-à-dire apte à et configuré pour transmettre les données, i.e. mesures, qu'il opère, par exemple via un système émetteur intégré, par exemple en mode Bluetooth^{™} ou en Wifi. Il communique ses données, i.e. mesures de SpO₂ et eventuellement de fréquence cardiaque, avec le débitmètre 30 communicant.

Par ailleurs, le débitmètre 30 communicant est configuré pour fournir de l'oxygène gazeux au patient à un débit donné. Typiquement, il comprend un passage de gaz interne, tel un conduit, véhiculant l'oxygène provenant de la source d'oxygène 40. Afin de déterminer le débit d'oxygène et permettre une détermination d'autres paramètres, comme la fréquence respiratoire du patient et/ou de la quantité d'oxygène fournie au patient chaque jour par exemple, on peut utiliser un ou des capteurs de pression agencés dans le boitier du débitmètre 30 permettent alors de mesurer la pression dans le passage et les variations de pression qui s'y opèrent. Des moyens de calcul, également agencés dans le boitier, permettent de calculer le débit à partir de ces mesures de pression, voire les autres paramètres utiles. Les moyens de calcul comprennent un microprocesseur, de préférence agencé sur une carte électronique, mettant en œuvre un ou des logiciels de calcul. Le débitmètre 30 est lui-aussi communicant, c'est-à-dire qu'il comprend des moyens d'émission/réception de données. Des moyens de fourniture de courant électrique, telle une pile ou batterie, rechargeable ou à usage unique, alimente les composants du débitmètre 30 nécessitant de l'électricité pour fonctionner, en particulier les moyens de calcul et le ou les capteurs. Un dispositif de ce type est par exemple décrit par EP-A-2291211.

Le débitmètre 30 peut comprendre aussi un afficheur configuré pour afficher des informations utiles, par exemple le débit d'oxygène délivré par le débitmètre 30.

Le débitmètre 30 peut comprendre en outre des moyens de calcul coopérant avec des moyens d'ajustement de débit

La source d'oxygène 40, à savoir ici une canalisation d'amenée d'oxygène gazeux, est relié fluidiquement au débitmètre 30 communicant, via une prise murale de distribution d'oxygène dans le mode de réalisation de la Figure 1, pour lui fournir un flux d'oxygène qui est distribué ensuite au patient, par exemple au moyen d'une interface respiratoire patient comme un masque respiratoire nasal ou facial, ou des canules respiratoires.

Par ailleurs, l'installation 1 de l'invention comprend aussi un dispositif d'affichage de données 50 configuré pour afficher des données fournies par la plateforme informatique de management de données 10, typiquement le dispositif d'affichage de données 50 peut être un ordinateur fixe ou portable, une tablette numérique ou un téléphone mobile multifonction (i.e. smartphone). Ce dispositif d'affichage de données 50 permet d'afficher des données, informations, alertes.

Comme déjà indiqué, afin d'assurer une transmission des données, c'est-à-dire des émissions et/ou réceptions de données, entre les différents composants de l'installation 1, il reste prévu des moyens de communication 60 configurés pour assurer ces échanges de données. Les moyens de communication 60 comprennent des moyens d'émission et/ou de réception de données, notamment en mode Bluetooth^{™}, Wifi, GSM , 4G/5G ou autre, et peuvent utiliser le réseau internet, i.e. web.

Les échanges de données ont lieu notamment, d'une part, depuis le dispositif de mesure de paramètres physiologiques 20 et le débitmètre 30 communicant vers la plateforme informatique de management de données 10 et, d'autre part, depuis la plateforme informatique de management de données 10 vers le dispositif d'affichage de données 50.

Par ailleurs, le débitmètre 30 peut aussi être configuré pour communiquer avec le dispositif de mesure de paramètres physiologiques 20, c'est-à-dire qu'ils peuvent être communicants, c'est-à-dire connectés entre eux. De même, pour ce qui est de la source d'oxygène 40 qui peut communiquer directement avec la plateforme informatique de management de données 10, comme illustré en Figure 1. A cette fin, ces équipements sont équipés de moyens d'émission et/ou de réception de données, de préférence en mode Wifi, Bluetooth^{™} et/ou via le réseau GSM et/ou internet ou autre.

Selon le mode de réalisation considéré, des échanges peuvent avoir lieu aussi dans l'autre sens, notamment de la plateforme informatique de management de données 10 vers le débitmètre 30 communicant pour par exemple.

On peut aussi prévoir que le dispositif d'affichage de données 50 soit doté de moyens d'entrée de données, tel le clavier numérique d'une tablette numérique ou un téléphone mobile multifonction, ou le clavier mécanique d'un ordinateur fixe ou portable, permettant de rentrer, changer ou modifier une (ou des) donnée affichée ou un paramètre donné, par exemple de modifier à distance le débit d'oxygène délivré par le débitmètre 30. Ainsi, un médecin qui constaterait une SpO₂ trop basse, par exemple après déclenchement d'une alerte visible sur le dispositif d'affichage de données 50, comme expliqué ci-après, pourrait décider de modifier à distance le débit d'oxygène afin de faire remonter la SpO₂ du patient. Il le ferait alors en fixant la valeur de débit désiré via les moyens d'entrée de données, i.e. clavier, et cette valeur de débit serait alors transmise au débitmètre 30 communicant par l'intermédiaire de la plateforme informatique de management de données 10. Le débitmètre 30 pourrait alors modifier ou moduler le débit d'oxygène le traversant, par exemple via la commande d'une vanne proportionnelle, pour augmenter le débit jusqu'à atteindre la valeur de débit fixée par le médecin.

Les moyens d'entrée de données peuvent aussi servir à configurer une ou des alarmes ou alertes, notamment des alertes relatives à la SpO2, à la fréquence respiratoire ou cardiaque ou au débit d'O₂.

De façon analogue, comme illustré en Figure 2, lorsque la source d'oxygène 40 est une bouteille de gaz portant un robinet de distribution de gaz, tel un RDI ou robinet à détendeur intégré, équipé d'un dispositif électronique communicant servant à mesurer la pression et/ou d'autonomie en gaz, et en général protégé par un capotage de protection, la source d'oxygène 40 peut aussi communiquer avec le dispositif de mesure de paramètres physiologiques 20. Pour ce faire, là encore, des moyens d'émission et/ou de réception de données, de préférence en mode Bluetooth^{™} ou wifi, sont embarqués par exemple dans le dispositif électronique communicant servant à mesurer la pression et/ou d'autonomie en gaz ou dans le capotage de protection protégeant le robinet de distribution de gaz. Les moyens d'émission et/ou de réception de données permettent d'assurer un échange de données avec le débitmètre 30, comme illustré en Figure 2.

Bien entendu, la bouteille de gaz peut être équipée d'un robinet de distribution de gaz plus simple, par exemple sans dispositif électronique, mais munie uniquement d'un manomètre à aiguille par exemple.

La source d'oxygène 40 de qualité médicale est un réseau de distribution de gaz médicaux classique, lorsque le patient de la maison de repos est dans sa chambre, c'est-à-dire stationnaire Lorsqu'il décide de sortir de sa chambre et donc de déambuler, la source d'oxygène 40 est alors une bouteille d'oxygène sous pression, de préférence équipée d'un RDI. Ces sources d'oxygène 40 peuvent fournir un débit d'oxygène allant jusqu'à au moins 15 I/min, ce qui est suffisant pour les patients, y compris ceux souffrant de détresse respiratoire sévère.

Plus généralement, le débitmètre 30 permet de fournir un débit d'oxygène de 0 à 30 L/min environ, par exemple plus de 15 L/min. Il est avantageusement entièrement digital avec une connectivité Wifi et/ou Bluetooth. Il comprend une mémoire interne de stockage de données, e.g. mesures, ayant une capacité importante pour le stockage des données, c'est-à-dire de plusieurs Go, voire plusieurs dizaines ou centaines de Go. Il est connecté au réseau de distribution d'O₂ 40 (cf. Fig. 1) et au réseau électrique en utilisation stationnaire, alors qu'en utilisation mobile, il est connecté à la bouteille d'oxygène (cf. Fig. 2), comme expliqué ci-avant, et embarque une batterie intégrée qui lui garantit une autonomie d'au moins 12 heures.

En fonction du mode de réalisation, un débitmètre 30 utilisable dans le cadre de l'invention peut avoir en outre les fonctionnalités suivantes optionnelles :
- il est configuré pour fonctionner selon un mode dit « confort », dans lequel le débit d'oxygène délivré va de 0 à 5 l/min. Dans ce mode, il délivre de l'oxygène à la demande, c'est-à-dire uniquement quand le patient inspire, c'est-à-dire pendant les phases inspiratoires du patient.
- il est configuré pour fonctionner selon un mode dit « continu », dans lequel le débit d'oxygène délivré est supérieur à 5 L/min. Un réglage fin par incrément par exemple de 0,5 l/min permet d'ajuster le débit désiré et ce, jusqu'à 30 L/min par exemple.
- il est configuré pour déterminer la fréquence respiratoire du patient, lorsqu'une canule nasale est utilisée pour fournir l'oxygène au patient et ce, que le patient soit sous oxygénothérapie en mode confort ou continu.
- il est configuré pour stocker ses propres données, notamment les données de mesures ou calculées, telles que fréquence respiratoire, consommation d'O₂, réglages de débit...., mais aussi des données provenant par exemple du saturomètre 20, tel que valeurs de SpO₂ et de rythme cardiaque, collectées pour un patient stationnaire ou mobile, c'est-à-dire déambulant. Il transmet tout ou partie des données à la plateforme informatique de management de données 10.
- il est configuré pour intégrer une fonction optionnelle de « surveillance de bouteille d'oxygène » en mode mobile (Fig. 2). Si la bouteille d'oxygène intègre dispositif électronique ayant une connectivité Bluetooth^{™} ou wifi par exemple, le débitmètre 30 peut également se connecter à ce dispositif électronique équipant la bouteille et envoyer les données qui en proviennent, en particulier l'autonomie en gaz et/ou la pression résiduelle, à la plateforme informatique de management de données 10.
- il est en outre configuré pour intégrer une fonction « d'oxygénothérapie à distance ». Un médecin qui ne se trouve pas sur site, c'est-à-dire qui n'est pas physiquement présent dans la maison de retraite ou analogue, peut déterminer un niveau de saturation (SpO₂) souhaité et définir à distance le débit d'O₂ à fournir au patient. Le débit peut s'afficher sur un afficheur du débitmètre. Ce débit suggéré est alors réglé par le personnel de la maison de retraite, sur le débitmètre 30.

Plus généralement, pendant une oxygénothérapie, le débitmètre 30 envoie en continu tout ou partie des données qu'il collecte ou qu'il mesure, en particulier SpO₂, rythme cardiaque, fréquence respiratoire, réglages de débit...., à la plateforme informatique de management de données 10. La plateforme informatique de management de données 10 met en œuvre un ou des algorithmes permettant de traiter et analyser ces données et préférentiellement d'identifier des tendances des données du patient et de prévoir des événements. Ces tendances peuvent ensuite être transmises au dispositif d'affichage de données 50 (ordinateur, téléphone, tablette numérique...) qui est configuré pour les afficher à l'attention d'un médecin par exemple. Le médecin peut alors les consulter, par exemple sous forme de valeurs (%), de courbes de tendance et/ou de tableaux de bord permettant de visualiser l'état, l'historique et l'évolution du patient sur une période de temps donnée.

Le médecin peut aussi recevoir des alertes précoces lorsque les données d'un patient évoluent dans une mauvaise direction, par exemple si sa saturation en oxygène reste ou devient trop faible, e.g. SpO2 < 92 % (seuil bas) pour un patient atteint de Covid19, ou à l'inverse trop forte, par exemple >98% (seuil haut). Comme déjà expliqué, le médecin peut alors modifier à distance le débit d'oxygène délivré par le débitmètre 30, sans avoir se rendre physiquement dans la maison de retraite, et sans intervention du personnel de la maison de retraite, ou alors peut envoyer au personnel de la maison de retraite, un débit à régler sur le débitmètre 30.

Selon un autre mode de réalisation, la plateforme informatique de management de données 10 peut aussi être configurée pour modifier ou corriger automatiquement le débit O₂ fourni par le débitmètre 30, c'est-à-dire sans passer par un médecin. Cette solution est totalement automatisée.

La plateforme informatique de management de données 10 comprend aussi ou coopère, e.g. communique, avec des moyens de stockage de données 70, tel un serveur de stockage ou analogue, permettant d'enregistrer les données brutes ou traitées par la plateforme informatique de management de données 10 et plus généralement, toutes les informations concernant la thérapie du patient, telle que sa consommation d'oxygène, sa fréquence respiratoire, son rythme cardiaque....,

Les données du patient peuvent éventuellement être aussi envoyées vers et stockées dans son dossier médical personnalisé accessible à distance, par exemple stocké sur un serveur d'une Caisse d'Assurance Maladie ou analogue.

Les analyses de données avancées opérées par la plateforme informatique de management de données 10 permettent notamment de détecter des tendances et de prévoir des événements susceptibles d'affecter le patient, notamment de prévenir une détérioration de son état de santé, par exemple si l'on constate une augmentation de sa fréquence respiratoire ou de sa consommation d'oxygène, ce qui permet une intervention rapide et/ou une modification précoce de la thérapie qui lui est appliquée.

Les données du patient peut être préférentiellement visualisées, i.e. affichées, sous forme de tableaux de bord dynamiques, i.e. mis à jour en temps réel, et visualisant par ailleurs les historiques et les évolutions de son traitement par oxygénothérapie. Des événements spécifiques peuvent être aussi enregistrés et des notifications et des alarmes en lien avec ces événements spécifiques peuvent être envoyées au médecin et personnel soignant par exemple.

A côté des informations concernant le patient, le personnel de la maison de retraite ou analogue, peut aussi consulter, via la plateforme informatique de management de données 10, des informations relatives à la source d'O₂ 40, comme par exemple l'autonomie et/ou la pression du réservoir... Ces informations sont transmises par la bouteille de gaz (cf. Fig. 2) et reçues par la plateforme informatique de management de données 10, comme expliqué ci-avant.

## Revendications

1. Installation (1) de fourniture d'oxygène à un patient permettant d'assurer une surveillance et un suivi dudit patient à distance comprenant :
- une plateforme informatique de management de données (10) configurée pour traiter des données,
- un dispositif de mesure de paramètres physiologiques (20) du patient configuré pour mesurer au moins la saturation en oxygène (SpO₂) du patient,
- un débitmètre (30) communicant configuré pour fournir de l'oxygène gazeux au patient à un débit donné, ledit débitmètre (30) comprenant en outre des moyens de détermination de la fréquence respiratoire du patient et/ou de la quantité d'oxygène fournie au patient,
- une source d'oxygène (40) relié fluidiquement au débitmètre (30) communicant pour lui fournir de l'oxygène,
- un dispositif d'affichage de données (50) configuré pour afficher des données fournies par la plateforme informatique de management de données (10), et
- des moyens de communication (60) configurés pour assurer des transmission de données au moins:
∘ depuis le dispositif de mesure de paramètres physiologiques (20) et le débitmètre (30) communicant vers la plateforme informatique de management de données (10), et
∘ depuis la plateforme informatique de management de données (10) vers le dispositif d'affichage de données (50).

2. Installation selon la revendication 1, **caractérisée en ce que** la plateforme informatique de management de données (10) est configurée pour traiter des mesures provenant du dispositif de mesure de paramètres physiologiques (20).

3. Installation selon l'une des revendications 1 ou 2, **caractérisée en ce que** le dispositif de mesure de paramètres physiologiques (20) du patient configuré pour mesurer en outre la fréquence cardiaque du patient.

4. Installation selon l'une des revendications 1 à 3, **caractérisée en ce que** le dispositif de mesure de paramètres physiologiques (20) comprend un saturomètre, de préférence connecté.

5. Installation selon la revendication 1, **caractérisée en ce que** le dispositif d'affichage de données (50) comprend un ordinateur fixe ou portable, une tablette numérique ou un téléphone mobile multifonction.

6. Installation selon l'une des revendications 1 ou 2, **caractérisée en ce que** la plateforme informatique de management de données (50) est configurée pour :
a) comparer des mesures de saturation en oxygène (i.e. SpO₂) provenant du dispositif de mesure de paramètres physiologiques à au moins une valeur-seuil haut et/ou une valeur-seuil bas de SpO₂ préfixée, et
b) déclencher une alerte visuelle et/ou sonore lorsque la saturation en oxygène (SpO₂) mesurée descend en dessous (i.e. est inférieure à) ladite valeur-seuil bas de SpO₂ préfixée ou excède (i.e. est supérieure à) ladite valeur-seuil haut de SpO₂ préfixée.

7. Installation selon la revendication 1, **caractérisée en ce que** les moyens de communication (60) comprennent des moyens d'émission et/ou de réception de données, notamment en mode Bluetooth^{™}, Wifi, GSM ou autre.

8. Installation selon l'une des revendications 1 ou 2, **caractérisée en ce que** la plateforme informatique de management de données (10) comprend un serveur informatique.

9. Installation selon l'une des revendications 1, 2 ou 8, **caractérisée en ce que** la plateforme informatique de management de données (10) comprend ou est reliée à des moyens de stockage de données (70).

10. Installation selon l'une des revendications 1 ou 2, **caractérisée en ce que** la source d'oxygène (40) comprend un récipient d'oxygène sous pression, telle une bouteille de gaz, ou une canalisation ou tuyau d'amenée d'oxygène.

11. Installation selon la revendication 1 ou 10, **caractérisée en ce que** le débitmètre (30) communicant est agencé entre la source d'oxygène (40) et une interface respiratoire patient reliée à la source d'oxygène (40) via au moins un conduit de gaz flexible.

12. Installation selon la revendication 1, **caractérisée en ce que** le dispositif de mesure de paramètres physiologiques (20) est configuré pour communiquer des données au débitmètre (30) ou à la plateforme informatique de management de données (10).

13. Installation selon la revendication 1, **caractérisée en ce que** le dispositif d'affichage de données (50) comprend en outre des moyens d'entrée de données permettant de rentrer, changer ou modifier une (ou des) donnée affichée ou un paramètre donné, par exemple de modifier à distance le débit d'oxygène délivré par le débitmètre, ou configurer une ou des alarmes ou alertes.

14. Installation selon la revendication 1, **caractérisée en ce que** le débitmètre (30) communicant comprend des moyens d'ajustement de débit pour ajuster ou changer le débit d'oxygène qu'il fournit au patient.

15. Installation selon la revendication 1, **caractérisée en ce que** la plateforme informatique de management de données (10) est en outre configurée pour modifier ou corriger automatiquement le débit O₂ fourni par le débitmètre (30).

## Revendications modifiées

### Revendications modifiées conformément à la règle 137(2) CBE.

1. Installation (1) de fourniture d'oxygène à un patient permettant d'assurer une surveillance et un suivi dudit patient à distance comprenant :
- une plateforme informatique de management de données (10) comprenant un serveur informatique, configurée pour traiter des données,
- un dispositif de mesure de paramètres physiologiques (20) du patient configuré pour mesurer au moins la saturation en oxygène (SpO₂) du patient,
- un débitmètre (30) communicant configuré pour fournir de l'oxygène gazeux au patient à un débit donné, ledit débitmètre (30) comprenant en outre des moyens de détermination de la fréquence respiratoire du patient et/ou de la quantité d'oxygène fournie au patient,
- une source d'oxygène (40) relié fluidiquement au débitmètre (30) communicant pour lui fournir de l'oxygène,
- un dispositif d'affichage de données (50) configuré pour afficher des données fournies par la plateforme informatique de management de données (10), et
- des moyens de communication (60) comprenant des moyens d'émission et/ou de réception de données, et configurés pour assurer des transmission de données au moins:
∘ depuis le dispositif de mesure de paramètres physiologiques (20) et le débitmètre (30) communicant vers la plateforme informatique de management de données (10), et
∘ depuis la plateforme informatique de management de données (10) vers le dispositif d'affichage de données (50),
et dans laquelle la plateforme informatique de management de données (10) est configurée pour traiter des mesures provenant du dispositif de mesure de paramètres physiologiques (20) et du débitmètre (30) communicant.

2. Installation selon la revendication 1, **caractérisée en ce que** le dispositif de mesure de paramètres physiologiques (20) du patient configuré pour mesurer en outre la fréquence cardiaque du patient.

3. Installation selon l'une des revendications 1 ou 2, **caractérisée en ce que** le dispositif de mesure de paramètres physiologiques (20) comprend un saturomètre connecté.

4. Installation selon la revendication 1, **caractérisée en ce que** le dispositif d'affichage de données (50) comprend un ordinateur fixe ou portable, une tablette numérique ou un téléphone mobile multifonction.

5. Installation selon la revendication 1, **caractérisée en ce que** la plateforme informatique de management de données (50) est configurée pour :
a) comparer des mesures de saturation en oxygène (i.e. SpO₂) provenant du dispositif de mesure de paramètres physiologiques à au moins une valeur-seuil haut et/ou une valeur-seuil bas de SpO₂ préfixée, et
b) déclencher une alerte visuelle et/ou sonore lorsque la saturation en oxygène (SpO₂) mesurée descend en dessous (i.e. est inférieure à) ladite valeur-seuil bas de SpO₂ préfixée ou excède (i.e. est supérieure à) ladite valeur-seuil haut de SpO₂ préfixée.

6. Installation selon la revendication 1, **caractérisée en ce que** les moyens de communication (60) comprennent des moyens d'émission et/ou de réception de données en mode Bluetooth^{™}, Wifi, GSM, 4G/5G ou autre.

7. Installation selon la revendication 1, **caractérisée en ce que** la plateforme informatique de management de données (10) comprend ou est reliée à des moyens de stockage de données (70).

8. Installation selon l'une des revendications 1, **caractérisée en ce que** la source d'oxygène (40) comprend un récipient d'oxygène sous pression, telle une bouteille de gaz, ou une canalisation ou tuyau d'amenée d'oxygène.

9. Installation selon la revendication 1 ou 8, **caractérisée en ce que** le débitmètre (30) communicant est agencé entre la source d'oxygène (40) et une interface respiratoire patient reliée à la source d'oxygène (40) via au moins un conduit de gaz flexible.

10. Installation selon la revendication 1, **caractérisée en ce que** le dispositif de mesure de paramètres physiologiques (20) est configuré pour communiquer des données au débitmètre (30) ou à la plateforme informatique de management de données (10).

11. Installation selon la revendication 1, **caractérisée en ce que** le dispositif d'affichage de données (50) comprend en outre des moyens d'entrée de données permettant de rentrer, changer ou modifier une (ou des) donnée affichée ou un paramètre donné ou configurer une ou des alarmes ou alertes.

12. Installation selon la revendication 1, **caractérisée en ce que** le débitmètre (30) communicant comprend des moyens d'ajustement de débit pour ajuster ou changer le débit d'oxygène qu'il fournit au patient.

13. Installation selon la revendication 1, **caractérisée en ce que** la plateforme informatique de management de données (10) est en outre configurée pour modifier ou corriger automatiquement le débit O₂ fourni par le débitmètre (30).

14. Installation selon la revendication 11, **caractérisée en ce que** le dispositif d'affichage de données (50) comprend en outre des moyens d'entrée de données permettant de modifier à distance le débit d'oxygène délivré par le débitmètre.
